# EUROPEAN PATENT SPECIFICATION

(11) **EP 4 228 740 B1**
(45) Date of publication and mention of the grant of the patent: **09.07.2025**
(21) Application number: 21879606.8
(22) Date of filing: 02.09.2021
(51) Int. Cl.: A61N 1/36, A61N 1/05, A61N 1/372

(54) **SELF-FITTING OF PROSTHESIS**
SELBSTANPASSUNG EINER PROTHESE
AUTOAJUSTEMENT DE PROTHÈSE

(30) Priority: 15.10.2020 US 202063092055 P
(43) Date of publication of application: 23.08.2023
(73) Proprietor: Cochlear Limited, Macquarie University, New South Wales 2109 (AU)
(72) Inventor: VAN DIJK, Bastiaan, Macquarie University, New South Wales 2109 (AU); QAZI, Obaid ur Rehman, Macquarie University, New South Wales 2109 (AU)
(74) Representative: Grünecker Patent- und Rechtsanwälte PartG mbB
(86) International application number: PCT/IB2021/058025
(87) International publication number: WO 2022/079506

(56) References cited:
- WO-A1-97/48447
- WO-A2-2006/136174
- KR-A- 20140 126 932
- US-A1- 2011 060 702
- US-A1- 2013 101 128
- US-A1- 2017 070 833
- US-A1- 2019 151 661
- US-B2- 8 666 503
- MEEUWS, M.: "Cochlear implant telemedicine: Remote fitting based on psychoacoustic self-tests and artificial intelligence", COCHLEAR IMPLANTS INTERNATIONAL, vol. 21, no. 5, 2 September 2020 (2020-09-02), GB, pages 260 - 268, XP009535780, ISSN: 1467-0100, DOI: 10.1080/14670100.2020.1757840

## Description

### BACKGROUND

Medical devices have provided a wide range of therapeutic benefits to recipients over recent decades. Medical devices can include internal or implantable components/devices, external or wearable components/devices, or combinations thereof (e.g., a device having an external device communicating with an implantable component). Medical devices, such as traditional hearing aids, partially or fully-implantable hearing prostheses (e.g., bone conduction devices, mechanical stimulators, cochlear implants, etc.), pacemakers, defibrillators, functional electrical stimulation devices, and other medical devices, have been successful in performing lifesaving and/or lifestyle enhancement functions and/or recipient monitoring for a number of years.

The types of medical devices and the ranges of functions performed thereby have increased over the years. For example, many medical devices, sometimes referred to as "implantable medical devices", now often include one or more instruments, apparatus, sensors, processors, controllers or other functional mechanical or electrical components that are permanently or temporarily implanted in a recipient. These functional devices are typically used to diagnose, prevent, monitor, treat, or manage a disease/injury or symptom thereof, or to investigate, replace or modify the anatomy or a physiological process. Many of these functional devices utilize power and/or data received from external devices that are part of, or operate in conjunction with, implantable components.

US20111060702 discloses a system comprising an auditory prosthesis.

### SUMMARY

The invention is defined by the independent claims. In a non-claimed example, there is a method comprising: after an initial clinical fitting of a sensory prosthesis to a recipient, performing automated fitting of the sensory prosthesis to the recipient; logging the automated fitting in a log; analyzing the log to determine fitting progress; determining that the fitting progress indicates lack of progress; and responsive to determining that the fitting progress indicates lack of progress, generating a clinician event.

In another example, there is a system comprising: an auditory prosthesis configured to provide stimulation to a recipient to cause auditory percepts based on a current map; and one or more processors configured to: receive an indication from the recipient regarding a quality of stimulation; modify the current map based on the quality of stimulation; determining a difference between the modified current map and a target map; and responsive the difference failing to satisfy a threshold, generate a clinician event.

In an example, there is a computer-readable medium having instructions stored thereon that, when executed by one or more processors cause the one or more processors to: after an initial clinical fitting of a sensory prosthesis to a recipient, perform automated fitting of the sensory prosthesis to the recipient, wherein to perform the automated fitting includes to: query the recipient regarding stimulation provided by the sensory prosthesis; receive a response to the querying from the recipient; and modifying a map of the sensory prosthesis based on the received response; determine fitting progress of the automated fitting; and responsive determining that the fitting progress indicates a lack of progress, generate a clinician event.

### BRIEF DESCRIPTION OF THE DRAWINGS

The same number represents the same element or same type of element in all drawings.
FIG. 1 illustrates an example sensory prosthesis fitting system that includes a sensory prosthesis that can benefit from the use of technologies described herein.
FIG. 2 illustrates a first example method for performing automated fitting.
FIG. 3 illustrates one or more processors configured to perform a second example method for performing fitting.
FIG. 4 illustrates instructions that, when executed by one or more processors cause the one or more processors to perform a third method that includes one or more operations for performing fitting.
FIG. 5, which is made up of FIGS. 5A, 5B, and 5C, illustrates a method for performing fitting.
FIG. 6 is a functional block diagram of an implantable stimulator system that can benefit from the technologies described herein.
FIG. 7 illustrates an example cochlear implant system that can benefit from use of the technologies disclosed herein.
FIG. 8 illustrates a retinal prosthesis system that comprises an external device, a retinal prosthesis and a mobile computing device.
FIG. 9 illustrates an example of a suitable computing system with which one or more of the disclosed examples can be implemented.

### DETAILED DESCRIPTION

Current sensory prosthesis fitting is driven by clinicians measuring threshold and comfort levels at every fitting session. After initial fitting of a prosthesis, levels tend to be unstable and recipients have difficulty identifying what they perceive (e.g., audibly or visually perceive). Thus, resources spent (e.g., clinical resources) measuring levels in the first session can be potentially inefficiently spent. By contrast, resources can be conserved by having the first fitting session be conducted to arrive at a map that gives comfortable sensing at a reasonable (though not perfect) level of intensity.

In an example, the initial or subsequent fitting is based on a set of maps (e.g., operating parameters for the sensory prosthesis) extracted from data analysis. The set of maps can fit typical evolution of map increases. In the first session one of these maps is transferred to the sensory prosthesis (e.g., without any measurements). Then, during the first two weeks of use, a sensory prosthesis application slowly and automatically increases the loudness of the map (e.g., increasing to different map profiles based on the data analysis). Increases are based on interventions at regular intervals (e.g. daily) and take into account: logs of use, trajectory compared to typical or expected trajectory, and answers of the user to questions posed by the sensory prosthesis application. In some examples, formal testing is also used. In addition to the automated fitting, the recipient (or a caregiver thereof) can instigate a map change when the map is too soft or the recipient experiences uncomfortable stimulation. The clinician can follow recipient progress during the fitting process via a cloud-based data sharing system. The sensory prosthesis can also automatically indicate problems (for instance the user never accepts a map change) and flag them to the clinician. Other problems (e.g., fitting not progressing as well as expected) can be used to trigger an alert to schedule a session with a clinician. Further, because of variations how recipients progress, the scheduling of a session can be customized based on actual progress during automated fitting rather than future prediction.

Thus, in some examples, the first fitting session is quick and easy, such as by including no measurements. Then, when the recipient comes back for the next clinical session they have a stable map and sensing experience, so subsequent fitting will be more efficient to optimize the map. The technology can be configured to recognize the range of variability among individuals, which applies even within groups who share a similar demography and/or perceiving history. Moreover, there are variations among an individual's ability to reliably self-report on their sensory precepts. The clinic management thus have a greater degree of confidence that the recipient can be given a cost-effective benefit from a clinical consultation.

### System

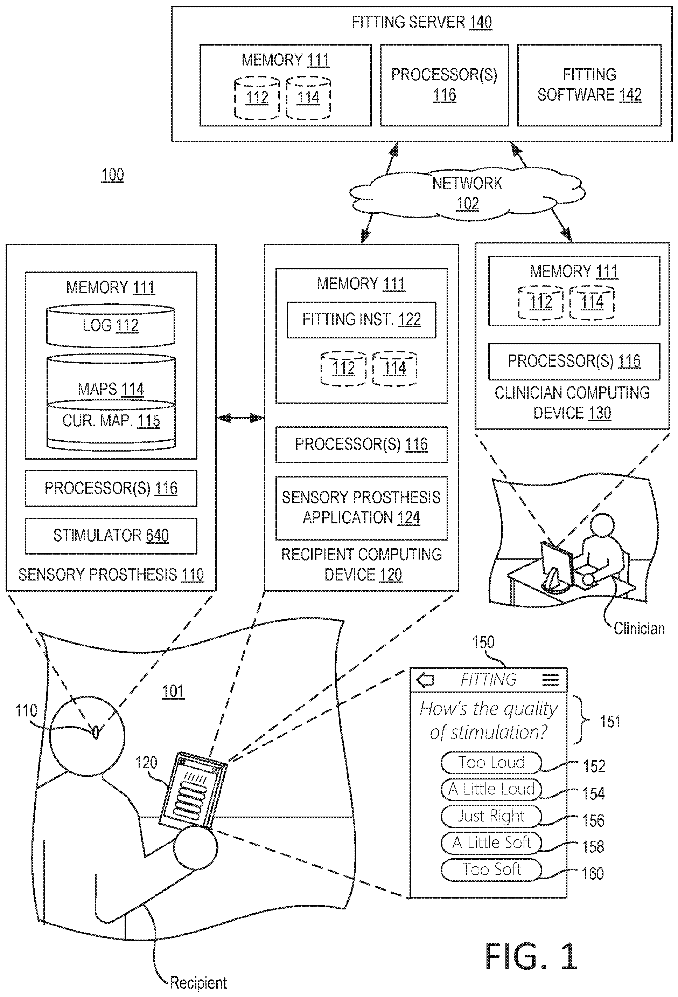

FIG. 1 illustrates an example sensory prosthesis fitting system 100 that includes a sensory prosthesis 110 that can benefit from the use of technologies described herein. The system 100 further includes a recipient computing device 120, a clinician computing device 130, and a fitting server 140, which are connected over a network 102. The network 102 is a computer network, such as the Internet, which facilitates the communication of data among computing devices connected to the computer network.

As illustrated, the sensory prosthesis 110 and the recipient computing device 120 are operated by the recipient in an environment 101. The environment 101 defines the conditions in which the sensory prosthesis 110 and the recipient computing device 120 operate. In many examples herein, the environment 101 includes the auditory conditions in which the sensory prosthesis 110 functions. Such auditory conditions can include, for example, a loudness of noise in the environment (e.g., whether the environment 101 is loud or quiet). Other examples relate to the visual environment in which the sensory prosthesis 110 functions. Such visual conditions can include, for example, brightness or colors of the environment.

The sensory prosthesis 110 is a medical apparatus relating to a recipient's sensory system. For example, where the sensory prosthesis 110 is an auditory prosthesis, the sensory prosthesis 110 can be configured to provide stimulation to a recipient to cause auditory percepts based on a current map 115 and audio detected in the environment 101. Where the sensory prosthesis is a visual prosthesis, the sensory prosthesis 110 can be configured to provide stimulation to a recipient to cause visual percepts based on a current map 115 and light detected in the environment 101.

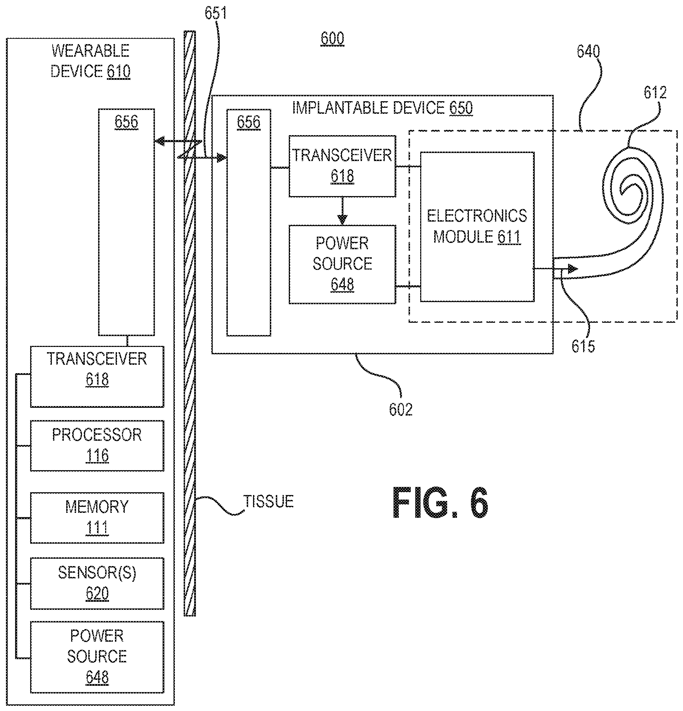

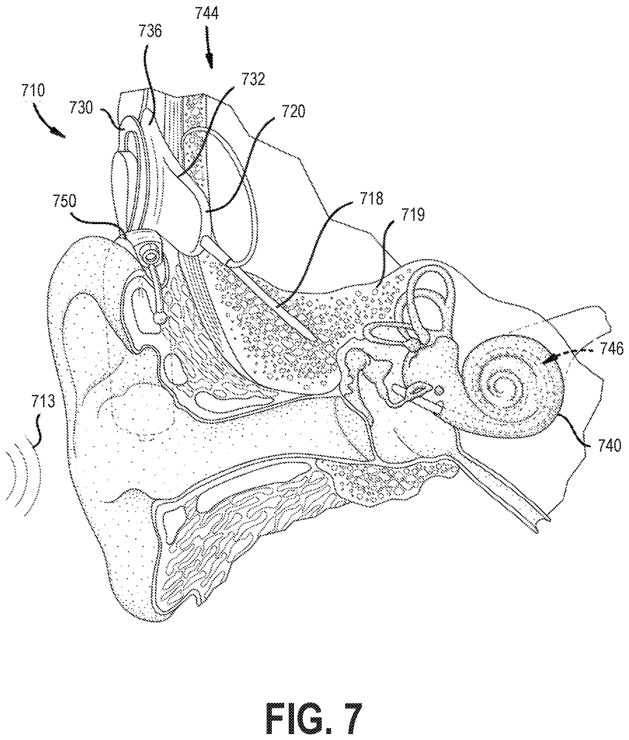

In an example, the sensory prosthesis 110 is an auditory prosthesis, such as a cochlear implant, bone conduction device (e.g., percutaneous bone conduction device, transcutaneous bone conduction device, active bone conduction device, and passive bone conduction device), or a middle ear stimulator, among others. The sensory prosthesis 110 can take any of a variety of forms and examples are such forms are described in more detail in FIG. 6 (showing a stimulator device) and FIG. 7 (showing a cochlear implant). In an example, the sensory prosthesis 110 is a visual prosthesis, such as a retinal prosthesis.

In the illustrated example, the sensory prosthesis 110 includes a memory 111, one or more processors 116, and a stimulator 640, among other components. In many examples, the sensory prosthesis 110 is a stimulator configured to cause the recipient to experience a sensory percept.

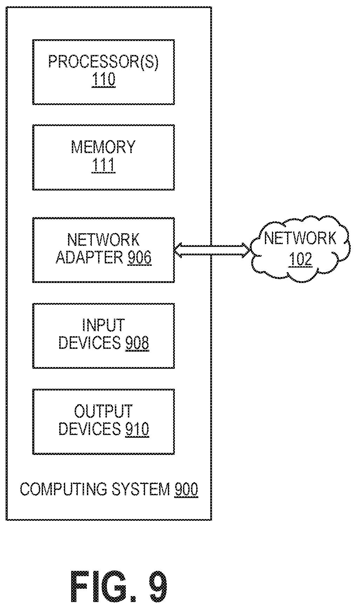

The memory 111 is one or more software- or hardware-based computer-readable storage media operable to store information accessible by the one or more processors 116. Additional details regarding the memory 111 are described in relation to FIG. 9. In the illustrated example, the memory 111 stores a log 112 and one or more maps 114.

The log 112 is a set of one or more data structures that are records of data, activity, or events relevant to the sensory prosthesis 110. In an example, the log 112 includes data regarding multiple fitting sessions. The one or more data structures of the log can be implemented in any of a variety of ways.

The maps 114 are one or more settings for the sensory prosthesis 110. In an example, the one or more maps 114 describes an allocation of frequencies from a filter bank or other frequency analyzer to individual electrodes of the stimulator 640. In an example, the one or more maps 114 describe electrical maps from sound levels in one or more or all of the frequency bands to electrical stimulation levels. The one or more maps 114 can be performed on a one-to-one basis, with each filter output is allocated to a single electrode. The one or more maps 114 can be created based on parameters, such as threshold levels (T levels) and maximum comfort levels (C levels) for one or more or all stimulation channels of the sensory prosthesis 110. In an example, the one or more maps 114 are stored by programming the sensory prosthesis 110 or by any other process that sets the channels of the sensory prosthesis 110 to have the map 114. Example maps and related techniques are described in US 2008/0119910 and US 9,757,562. Example maps are further described in the references incorporated below regarding fitting (see discussion of operation 210, below).

The maps 114 can each be or include one or more parameters having values that affect how the sensory prosthesis 110 operates. For instance, the maps 114 can include a map 114 having minimum and maximum stimulation levels for frequency bands of stimulation channels. The map 114 is then used by the sensory prosthesis 110 to control an amount of stimulation to be provided. For instance, where the sensory prosthesis 110 is a cochlear implant, the map 114 affects which electrodes of the cochlear implant to stimulate and in what amount based on a received sound input. In some examples, the maps 114 include two or more predefined groupings of settings selectable by the recipient. One of the two or more predefined groupings of settings may be a default setting. In an example, the maps 114 can be ordered, such as based on relative loudness of the maps. For example, a first map 114 can have a lower loudness than an nth map 114, where n is the highest numbered map 114. In some examples, the differences between the maps 114 are simply intensity of stimulation. In other examples, there can be other differences between maps 114. In some implementations, the maps 114 can have different shapes compared to one another. For instance, the maps can be based on principle component analysis.

The maps 114 can also include sound processing settings that modify sound input before it is converted into a stimulation signal. Such settings can include, for example, particular audio equalizer settings can boost or cut the intensity of sound at various frequencies. In examples, the maps 114 can include a minimum threshold for which received sound input causes stimulation, a maximum threshold for preventing stimulation above a level which would cause discomfort, gain parameters, loudness parameters, and compression parameters. The maps 114 can include settings that affect a dynamic range of stimulation produced by the sensory prosthesis 110. As described above, many of the maps 114 affect the physical operation of the sensory prosthesis 110, such as how the sensory prosthesis 110 provides stimulation to the recipient in response to sound input received from the environment 101.

The one or more processors 116 include one or more hardware or software processors (e.g., microprocessors or central processing units). In many examples, the one or more processors 116 are configured to obtain and execute instructions from the memory 111. Additional details regarding the one or more processors 116 are described in relation to FIG. 9.

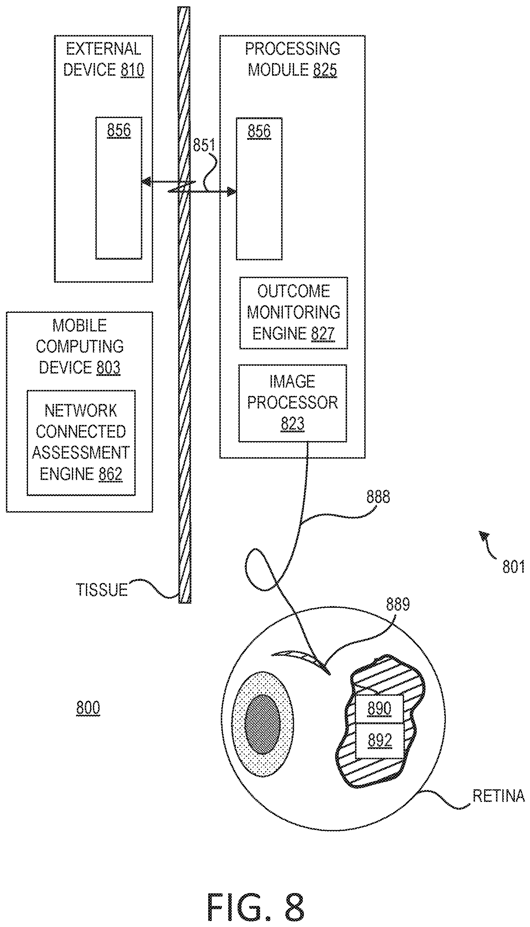

The stimulator 640 includes the stimulation generation and delivery components as well as additional support components of the sensory prosthesis 110. Examples include an electronics module and stimulator assembly as described in more detail in FIG. 6, the stimulator unit and elongate lead as described in more detail in FIG. 7, and the sensor-stimulator of FIG. 8. As a specific example, the stimulator 640 is or includes an auditory stimulator. The auditory stimulator can be a component configured to provide stimulation to a recipient's auditory system to cause a hearing percept to be experienced by the recipient. Examples of components usable for auditory stimulation include components for generating air-conducted vibrations, components for generating bone-conducted vibration, components for generating electrical stimulation, other components, or combinations thereof.

The recipient computing device 120 is a computing device associated with the recipient of the sensory prosthesis 110. In many examples, the recipient computing device 120 is a cell phone, tablet, laptop, smart watch, or heart rate monitor, but can take other forms. As illustrated, the recipient computing device 120 includes memory 111 and one or more processors 116.

As illustrated, the memory 111 includes fitting instructions 122. The fitting instructions 122 can be instructions executable by the one or more processors 116 of the recipient computing device 120 to implement one or more methods or operations described herein. In some examples, the fitting instructions 122 are a part of instructions executable to provide a sensory prosthesis application 124. In some examples, the memory 111 stores the log 112 and one or more maps 114.

In examples, the recipient computing device 120 includes or implements the sensory prosthesis application 124 that operates on the recipient computing device 120 and cooperates with the sensory prosthesis 110. For instance, the sensory prosthesis application 124 can control the sensory prosthesis 110 (e.g., based on input received from the recipient) and obtain data from the sensory prosthesis 110. The recipient computing device 120 can connect to the sensory prosthesis 110 using, for example, a wireless radiofrequency communication protocol (e.g., BLUETOOTH). The sensory prosthesis application 124 transmits or receives data from the sensory prosthesis 110 over such a connection. The sensory prosthesis application 124 can also stream audio to the sensory prosthesis 110, such as from a microphone of the recipient computing device 120 or an application running on the recipient computing device 120 (e.g., a video or audio application).

In some examples, the sensory prosthesis application 124 provides a fitting user interface 150. The fitting user interface 150 is a user interface configured to obtain fitting information from the recipient. As illustrated, the fitting user interface 150 includes a query 151 to the user in the form of a text prompt and five user interface elements (e.g., buttons) selectable by the user and configured to obtain input from the recipient. As illustrated, the fitting user interface 150 includes a first user interface element 152 selectable to indicate that the stimulation is too loud, a second user interface element 154 selectable to indicate that the stimulation is a little loud, a third user interface element 156 selectable to indicate that the stimulation is just right, a third user interface element 158 selectable to indicate that the stimulation is a little soft, and a fifth user interface element 160 selectable to indicate that the stimulation is too soft. Other implementations of the user interface 150 are also usable. For example, slider user interface elements, drop down menus, and other systems can be used to receive input from the user.

The clinician computing device 130 is a computing device used by a clinician. A clinician is a medical professional, such as an audiologist. In an example, the clinician is a medical professional that provides care or supervision for the recipient. The clinician computing device 130 includes one or more software programs usable to monitor the sensory prosthesis 110, such as fitting progress thereof. The clinician computing device 130 can include memory 111 and one or more processors 116. In an example, the memory stores instructions that, when executed by the one or more processors 116 causes the one or more processors 116 to obtain data regarding fitting of the sensory prosthesis 110 (e.g., via the server 140 or by a direct connection between the sensory prosthesis 110 or the recipient computing device 120 and the clinician computing device 130) and present such data to the clinician over a clinician user interface. In some examples, the data includes data stored in the log 112.

The fitting server 140 is a server computing device remote from the sensory prosthesis 110, recipient computing device 120, and the clinician computing device 130. The fitting server 140 is communicatively coupled to the recipient computing device 120 and the clinician computing device 130. In many examples, the fitting server 140 is indirectly communicatively coupled to the sensory prosthesis 110 through the recipient computing device 120 (e.g., via the sensory prosthesis application 124). In some examples, the fitting server 140 is directly communicatively coupled to the sensory prosthesis 110. The fitting server 140 includes memory 111, one or more processors 116, and fitting software 142. The fitting software 142 is software operable to perform one or more operations described herein, such as operations that fit the sensory prosthesis 110. The fitting software 142 can customize the sensory prosthesis 110 based on feedback from the recipient or the clinician.

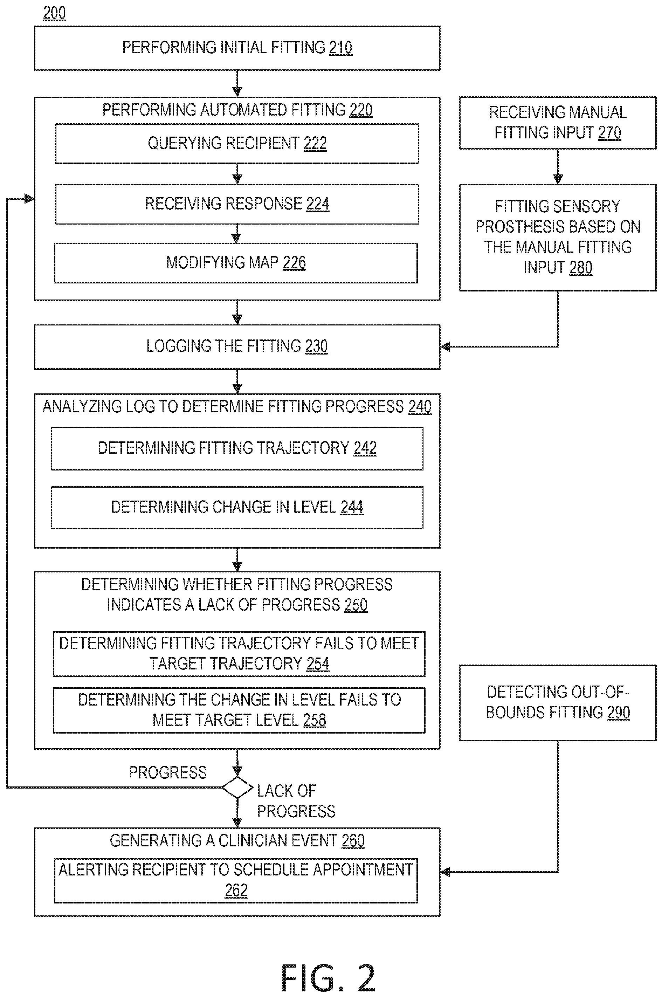

The components of the system 100 can cooperate to perform one or more methods that improves the performance of the sensory prosthesis 110, such as by fitting the sensory prosthesis 110 and generating one or more clinician events. An example of such a method are described below in relation to FIGS. 2-5C.

### First Method

FIG. 2 illustrates a first non-claimed example method 200. In an example, the method 200 is partially or wholly performed by the sensory prosthesis 110. In an example, the method 200 is partially or wholly performed by the recipient computing device 120 communicatively coupled to the sensory prosthesis 110.

Operation 210 includes performing an initial fitting. During the initial fitting, the sensory prosthesis 110 is tailored, customized, or otherwise adjusted for the specific needs, wants, or characteristics of the recipient of the sensory prosthesis 110. For example, the initial fitting can be performed or led by a clinician at a clinic. In some examples, the initial fitting can be performed by a software system at a clinic. The initial fitting can produce one or more maps. Example fitting software includes CUSTOM SOUND PRO by COCHLEAR. Example techniques for fitting that can be used with techniques described herein are described at least at US 2018/0275956, US 2018/0256895, US 2016/0337768, US 2016/0158547, US 2015/0343217, and US 2015/0237452. In some examples, the initial fitting is the first time the sensory prosthesis 110 is fit to the recipient. In other examples, initial fitting can refer to a clinician-led fitting that occurs at a clinic. The initial fitting can be initial with respect to subsequent automated fitting and can act as a baseline fitting on which the subsequent fitting is based. Where the initial fitting is the first time the sensory prosthesis 110 is fit to the recipient, an initial map 114 may have relatively low intensity (e.g., to permit a recipient to become accustomed to artificial stimulation by the sensory prosthesis 110).

Operation 220 includes performing automated fitting. For example, after the initial clinical fitting of the sensory prosthesis 110 to the recipient in operation 210, the automated fitting of the sensory prosthesis 110 to the recipient can be performed. In an example, the automated fitting is performed by the sensory prosthesis 110, the recipient computing device 120, the clinician computing device 130 (e.g., remotely), the fitting server 140 (e.g., remotely, or combinations thereof (e.g., the sensory prosthesis 110 and the recipient computing device 120 cooperate to accomplish the automated fitting). The automated fitting can be performed, for example, outside of the clinic where the initial fitting took place. In an example, the automated fitting can be automated in the sense that the automated fitting is directed or led by an automated process (e.g., performed by fitting software) rather than directly led by a clinician. As described in more detail herein, the automated fitting can include manual input from the recipient (or a caregiver for the recipient). Where manual fitting input is received, the fitting of the sensory prosthesis 110 can be based on the manual fitting input. As described below, the automated fitting can include operations 222, 224, and 226.

Operation 222 includes querying the recipient, such as regarding stimulation provided by the sensory prosthesis 110. For example, the user interface 150 is provided by the recipient computing device 120 to provide a query 151. In the illustrated example, the query 151 is regarding the quality of the stimulation. The query can be 151 rephrased in any of a variety of forms. Generally, the query 151 is configured to elicit a response from the recipient that is useful for fitting the sensory prosthesis 110. The querying can further include providing one or more user interface elements selectable by the recipient to provide a response (see, e.g., user interface elements 152, 154, 156, 158, and 160. Other input mechanisms can be presented or activated, such as a text box configured to receive text input from a user or by activating a microphone to receive voice input.

Operation 224 includes receiving a response to the querying. In an example, the response is received by detecting actuation of one or more of the user interface elements 152, 154, 156, 158, and 160. In other examples, the response from the recipient can be received through another form, such as free text entry or via voice (e.g., which is then converted using a voice-to-text system into text) on which natural language processing is performed to understand the input.

Operation 226 includes modifying a map 114 of the sensory prosthesis 110 based on the received response. In an example, the operation can include changing one or more properties of the current map 115. In another example, different map 114 of the maps 114 can be selected as the current map 115. For instance, the modification can include changing the map 114 from a first map 114 stored on the sensory prosthesis 110 to a second map 114 stored on the sensory prosthesis.

Operation 230 includes logging the fitting. For example, the operation 230 can include logging the automated fitting in a log 112. Logging can include storing data relevant to the fitting in the log 112. The relevant data can include the identity of the current map 115, the query 151 presented, the response to the query 151 that was received, the current date, the current time, the kind of environment 101 in which the sensory prosthesis 110 recently operated, other data, or combinations thereof. The logged data can further include data that can indicate a fitting progress. In some examples, the log 112 can be provided to different components of the system 100, such as the recipient computing device 120 and the server 140. In some examples, the recipient can view the log (or a visualization based on the log) using the sensory prosthesis application 124 (e.g., view the log locally or a remotely stored log).

Operation 240 includes analyzing the log 112 to determine fitting progress. For example, one or more statistical analyses can be performed in the data in the log 112. In an example, operation 240 includes operation 242 and operation 244.

Operation 242 includes determining fitting trajectory. The fitting trajectory can be one or more pieces of data or statistics that indicate how the automated fitting is progressing, has been progressing, or will progress. For example, the fitting trajectory can include data regarding changes to the mapping, such as a rate of change to the mapping (e.g., changes per week or per month). In a further example, the fitting trajectory can be a change in a dynamic range (e.g., the range between a threshold level and a comfort level) or rate of change in the dynamic range.

Operation 244 includes determining a change in level, such as a threshold level and a comfort level. In another example, the fitting trajectory can relate to change or rate of change in a particular characteristic of a map 114, such as a comfort level and/or a threshold level.

Operation 250 includes determining whether fitting progress indicates a lack of progress. In an example, the operation 240 can include operations 254, and 258.

Operation 254 includes determining the fitting trajectory fails to meet a target trajectory. For example, the target trajectory can be a predetermined trajectory set by the fitting system or the clinician. In some examples, the target trajectory is determined based on automatically or manually analyzing clinical maps. Starting stimulation levels can be compared with final (e.g., goal) stimulation levels and determine an expected (e.g., median) increase over time from the starting (or current) stimulation level to reach the final stimulation level. In some examples, the target trajectory is determined based on one or more audiograms (e.g., the audibility of sounds in free field), which can be a way to determine the suitability of minimum stimulation levels. In some examples, the target trajectory is determined based on objective measures, such as electrophysiological responses of the auditory nerve or the brain.

Determining that the fitting trajectory fails to meet the target trajectory can include comparing the determined trajectory and the target trajectory. In some examples, the target is customized to the recipient. The target can be algorithmically generated or manually specified by a clinician. In some examples, the target is based on how other similar recipients progressed over a particular period of time. In some examples, one or more aspects of the target trajectory can be based on a time series of audiograms or other psychophysical assessments of the hearing ability of the user.

Operation 258 includes determining a change in level fails to meet a target level. For example, the target level can be a target comfort level, such as may be predetermined (e.g., set automatically or by the clinician). The determining can include comparing the current level with the target to see if the current level surpasses or otherwise satisfies the target level.

Operation 260 includes generating a clinician event. For example, the operation 260 can be performed responsive to determining that the fitting progress indicates a lack of progress. In an example, the clinician event can be reporting a fitting status of the sensory prosthesis 110 to the clinician. In some examples, the clinician event includes to cause the clinician computing device 130 to generate an alert. In an example, the operation 260 includes operation 262. Operation 262 includes alerting the recipient to schedule an appointment, such as an appointment with a clinician. The appointment can be an appointment for a clinical fitting.

Operation 270 includes receiving manual fitting input. For example, the manual fitting input can be received outside of the automated fitting process (e.g., at a time when automated fitting is not occurring). The sensory prosthesis 110 can directly or indirectly (e.g., via the sensory prosthesis application 124) receive the manual fitting input. For example the sensory prosthesis application 124 can receive input from the recipient that activates a user interface (e.g., which can include one or more features of the user interface 150) over which the manual fitting input is received. The manual fitting input can include an input indicating that the stimulation provided by the sensory prosthesis 110 is undesirable to the recipient (e.g., by being too loud or too soft for a current or recent environment 101).

Operation 280 includes fitting the sensory prosthesis 110 based on the manual fitting input. Where the manual fitting input indicates that the stimulation perceived by the recipient is too soft, the current map 115 of the sensory prosthesis 110 can be changed to a map 114 that provides more intense stimulation. Where the manual fitting input indicates that the stimulation perceived by the recipient is too intense, the current map 115 of the sensory prosthesis 110 can be changed to a map 114 that provides less intense stimulation. The current map 115 can be changed using one or more of the techniques described above in relation to the automated fitting in operation 220.

Operation 290 includes detecting an out-of-bounds fitting. The out-of-bounds fitting can be a setting of a parameter or a mapping to a value that exceeds a maximum value or falls below a minimum value. For example, where the current map 115 is a loudest possible map 114 of the maps 114 (e.g., having the highest upper stimulation level), an attempt to increase the loudness of the current map 115 further can result in an out-of-bounds fitting. As another example, where the current map 115 is a softest possible map 114 of the maps 114 (e.g., having the lowest comfort level), an attempt to decrease the loudness of the current map 115 further can result in an out-of-bounds fitting. The out-of-bounds fitting can further be a fitting that violates one or more constraints, such as by attempting to set the comfort level lower than the threshold level. In an example, responsive to detecting the out-of-bounds fitting, the clinician event is generated. For instance, in response to detecting the out-of-bounds fitting, operation 260 is performed.

### Second Method

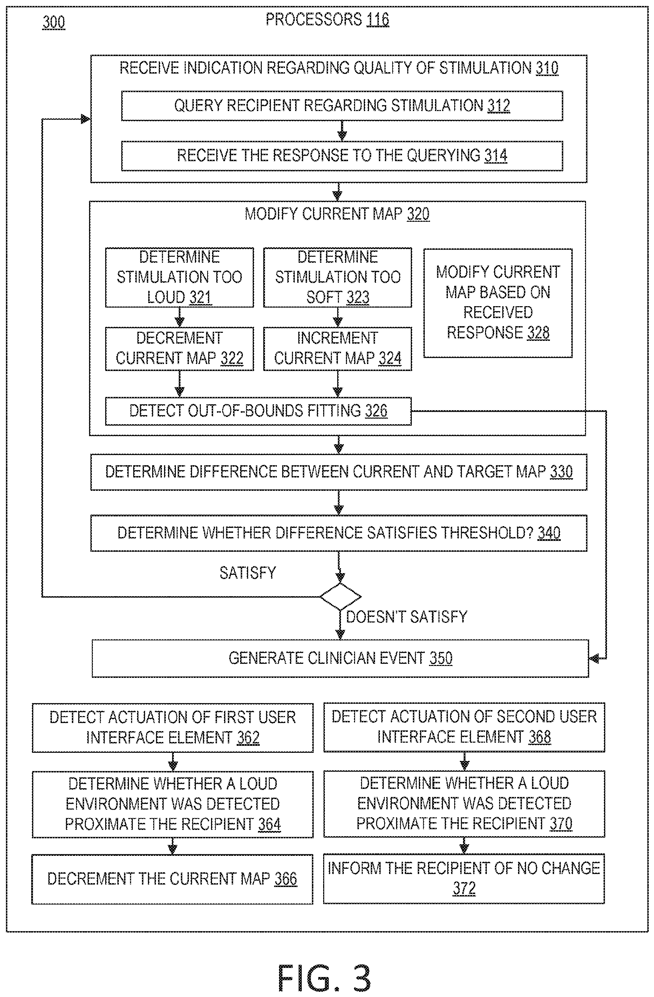

FIG. 3 illustrates one or more processors 116 configured to perform a second non-claimed example method 300. For example, the one or more processors 116 can be communicatively coupled to memory storing instructions that, when executed by the one or more processors 116, cause the one or more processors to perform the method 300. The one or more processors 116 can be processors of one or more of: the sensory prosthesis 110, the recipient computing device 120, the clinician computing device 130, or the fitting server 140.

Operation 310 includes to receive an indication regarding quality of stimulation. Quality of stimulation can include, for example ,comfort, acceptance, loudness, speech intelligibility, visual intelligibility, other qualities, or combinations thereof. For example, operation 310 can include operation 312 and operation 314. Operation 312 includes to query the recipient regarding stimulation. Where the sensory prosthesis 110 is an auditory prosthesis, the operation 312 can include querying the recipient regarding the stimulation provided by the auditory prosthesis. Where the sensory prosthesis 110 is a visual prosthesis, the operation 312 can include querying the recipient regarding the stimulation provided by the visual prosthesis. In an example, querying the recipient can include providing a user interface configured to receive input from the recipient. The user interface can be provided automatically or be manually accessed by the user (e.g., the recipient themselves or a caregiver of the recipient). For example, the operation 312 can include to provide a first user interface element 152 selectable to indicate that the stimulation is too loud, and provide a second user interface element 156 selectable to indicate that the stimulation is too soft. Additional example techniques that can be implemented are described above in operation 222.

Operation 314 includes receiving the response to the querying. For example, the response can be received from a user over a user interface provided in operation 312. Additional example techniques that can be implemented are described above in operation 224.

Operation 320 includes to modify a current map, such as based on the quality of stimulation. Example techniques that can be used to implement operation 320 are described above in operation 226. In an example, operation 320 includes operations 321, 322, 323, 324, 326, and 328.

Operation 321 includes to determine that stimulation is too loud (e.g., the intensity of stimulation is too high). For example, determining that the stimulation is too loud can be responsive to receiving input from a user (e.g., the recipient or a caretaker thereof) indicating that the stimulation is too loud. In addition or instead, determining that the stimulation is too loud can be determined based on activity of the recipient, such as the recipient operating the sensory prosthesis 110 with a low volume setting or attempting to lower the volume of the sensory prosthesis 110 (e.g., below a minimum volume).

Operation 322 includes to decrement a current map. For example, the operation 322 can be performed responsive to determining that the stimulation is too loud. Decrementing the current map 115 can include changing the current map 115 to a different map 114 that has an identifier that is one (or a different value) less than the identifier of the current map 115 (e.g., in pseudocode, current_map_id = current_map_id - 1).

Operation 323 includes to determine that the stimulation is too soft (e.g., the intensity of stimulation is too low). For example, determining that the stimulation is too soft can be responsive to receiving input from a user (e.g., the recipient or a caretaker thereof) indicating that the stimulation is too soft. In addition or instead, determining that the stimulation is too soft can be determined based on activity of the recipient, such as the recipient operating the sensory prosthesis 110 with a high volume setting or attempting to increase the volume of the sensory prosthesis 110 (e.g., beyond a maximum volume).

Operation 324 includes to increment the current map. For example, the operation 322 can be performed responsive to determining that the stimulation is too soft. Incrementing the current map 115 can include changing the current map 115 to a different map 114 that has an identifier that is one greater than the identifier of the current map 115 (e.g., in pseudocode, current_map_id = current_map_id + 1). The maps 114 can be associated with any of a variety of different identifiers. In some examples, incrementing (or decrementing) a map 114 can correspond to changing a program number. In other examples, the incrementing or decrementing of a map 114 does not correspond to changing a program number. In some examples, the maps 114 can be created using a technique (e.g., one or more data analysis techniques) and then the maps 114 are ranked. For example, the maps 114 can be ranked based on relative loudness, such that relatively higher ranked maps are relatively louder.

Operation 326 includes to detect an out-of-bounds fitting. For example, the operation 326 can include detecting an attempt to decrement the current map 115 beneath a map floor or attempting to increment the current map 115 above a map ceiling. For example, a sensory prosthesis 110 can include *n* maps 114 numbered from zero through *n* (inclusive). Detecting the out-of-bounds fitting can include attempting to change the current map 115 to a value less than zero or greater than *n*. Additional example techniques that can be implemented are described above in operation 290.

Operation 328 includes to modify the current map 115 based on a received response. Example techniques that can be implemented are described above in operation 226.

Operation 330 includes to determine a difference between a current map 115 and target map. For example, to determine the difference can include to determine a difference between an identifier of the current map 115 and an identifier of a target map. For instance, the target map 114 may be map 114 number seven and the current map 115 is map 114 number eight. Thus, the current map 115 is one greater than the target map. In another example, determining the difference between the current map 115 and the target map 114 can include comparing differences in parameters or other characteristics of the maps. For instance, a threshold or comfort level of the two maps is compared.

Operation 340 includes to determine whether the difference satisfies the threshold. For instance, the threshold can be the current map 115 being greater than or less than a predetermined threshold. In another example, the threshold can be the current map 115 having a particular characteristic that is greater than or less than a predetermined threshold.

Operation 350 includes to generate a clinician event. For example, the clinician event can be generated responsive to the difference failing to satisfy the threshold.

Operation 362 includes to detect actuation of a first user interface element. For example, a first user interface element 152 can be provided that is selectable to indicate that the stimulation is too loud.

Operation 364 includes to determine whether a loud environment was detected proximate the recipient. For example, it can be determined based on whether the loud environment was detected within a threshold amount of time. For instance, data regarding the environment 101 can be stored in the log 112 and the determining can be performed by analyzing the log 112.

Operation 366 includes to decrement the current map 115. For example, the current map 115 can be decremented responsive to the determining that the loud environment was detected proximate the recipient within the threshold amount of time. Decrementing the current map 115 can include changing the current map 115 to a different map 114 that has an identifier that is one (or a different value) less than the identifier of the current map 115.

Operation 368 includes to detect actuation of a second user interface element 156. For example, a second user interface element 156 can be provided that is selectable to indicate that the stimulation is too soft.

Operation 370 includes to determine whether the loud environment was detected proximate the recipient, such as is described in relation to operation 364.

Operation 372 includes to inform the recipient of no change. For example, responsive to the loud environment being detected proximate to the recipient within a threshold amount of time, the operation 372 can be performed.

### Third Method

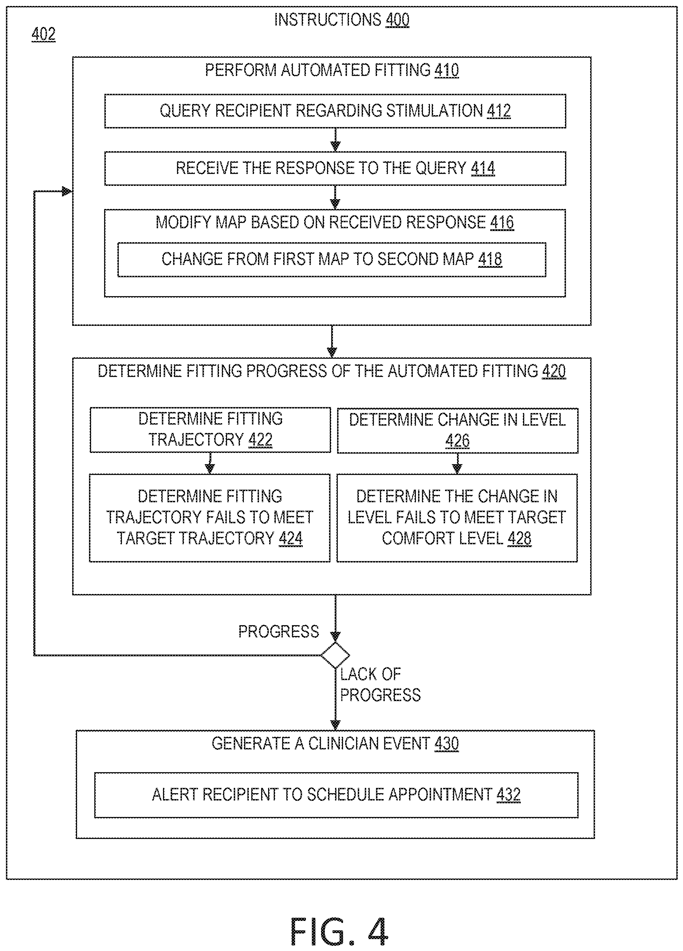

FIG. 4 illustrates instructions 400 that, when executed by one or more processors 116 cause the one or more processors 116 to perform a third non-claimed method 402 that includes one or more operations. The instructions 400 can be stored on a computer-readable medium that is a component of the sensory prosthesis 110, recipient computing device 120, clinician computing device 130, and the fitting server 140. In an example, the recipient computing device 120 is communicatively coupled to the sensory prosthesis 110.

Operation 410 includes to perform automated fitting. In an example, the operation 410 includes to perform after an initial clinical fitting of a sensory prosthesis 110 to a recipient, perform automated fitting of the sensory prosthesis 110 to the recipient. The operation 410 can include one or more aspects of operation 220, which describes performing automated fitting.

Operation 412 includes to query the recipient regarding stimulation provided by the sensory prosthesis 110. The operation 410 can include one or more aspects of operation 222, which describes querying the recipient.

Operation 414 includes to receive the response to the query from the recipient. The operation 414 can include one or more aspects of operation 224, which describes receiving a response to a query.

Operation 416 includes to modify the map 114 based on the received response. The operation 416 can include one or more aspects of operation 226, which describes modifying a map. In some examples, operation 416 includes operation 418. Operation 418 includes to change from a first map 114 to a second map 114. For example, the operation 418 can include changing the map 114 from a first map 114 stored on the sensory prosthesis 110 to a second map 114 stored on the sensory prosthesis. In other examples, the maps 114 can be stored elsewhere.

Operation 420 includes to determine fitting progress of the automated fitting. The operation 420 can include one or more aspects of operation 240, which describes determining fitting progress.

Operation 422 includes to determine a fitting trajectory. The operation 410 can include one or more aspects of operation 242, which describes determining a fitting trajectory.

Operation 424 includes to determine that the fitting trajectory fails to meet a target trajectory. The operation 410 can include one or more aspects of operation 254, which describes determining that the fitting trajectory fails to meet the target trajectory.

Operation 426 includes to determine a change in level. The operation 426 can include one or more aspects of operation 244, which describes determining a change in level.

Operation 428 includes to determine the change in level fails to meet a target level. The operation 426 can include one or more aspects of operation 258, which describes determining that the change in level fails to meet a target level.

Operation 430 includes to generate a clinician event. For example, the operation 430 can be performed responsive determining that the fitting progress indicates a lack of progress. The operation 430 can include one or more aspects of operation 260, which describes generating a clinician event. Operation 432 includes to alert a recipient to schedule an appointment. The operation 426 can include one or more aspects of operation 262, which describes alerting a recipient to schedule an appointment.

### Fourth Method

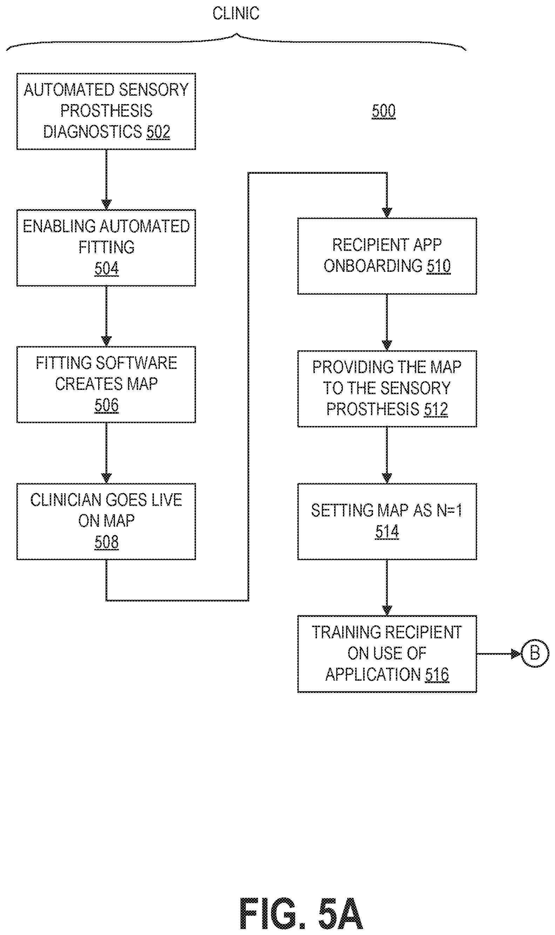

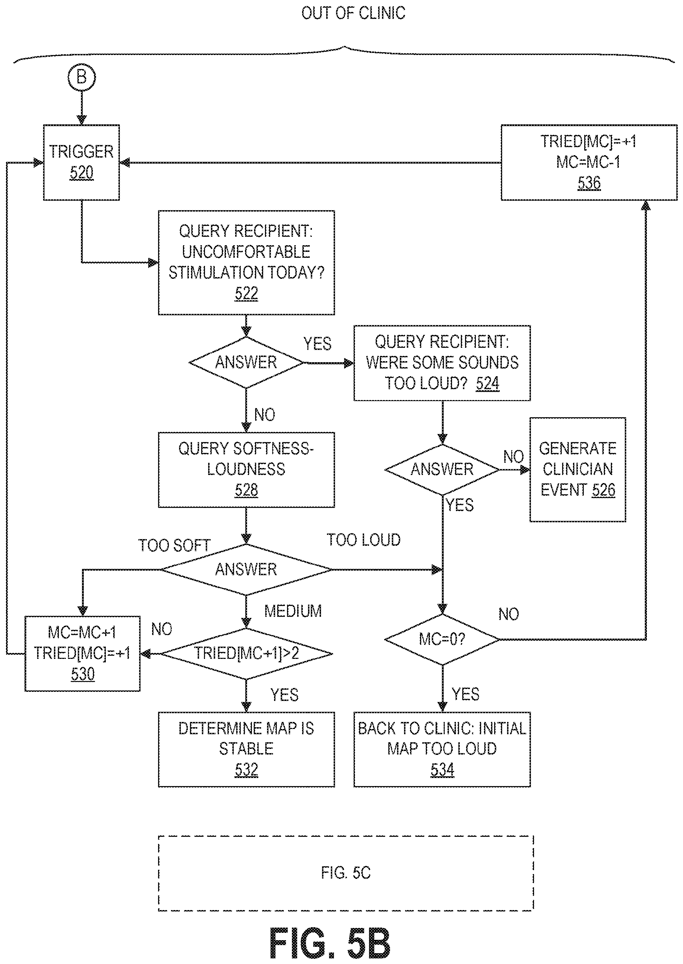

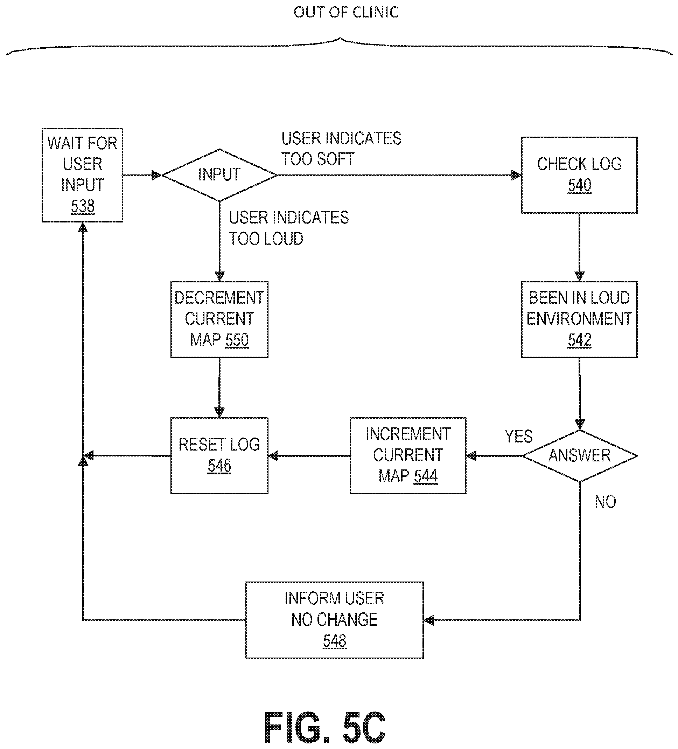

FIG. 5, which is made up of FIGS. 5A, 5B, and 5C, illustrates a non-claimed method 500. As shown in the figures, certain operations can be performed at a clinic and others can be performed out of the clinic (e.g., at home).

Operation 502 includes performing automated sensory prosthesis diagnostics. In an example, the automated sensory prosthesis diagnostics includes automated impedance check and electrode. The operation 502 can produce a log or report describing the status of various components of the sensory prosthesis 110. The log or report can facilitate diagnosing actual or potential faults with the device. Example automated diagnostics processes are described, for example, at U.S. Provisional Patent Application No. 62/904,069 (describing detecting neotissue formation proximate a sensory prosthesis), US 10,549,094 (describing detecting a physical state of a stimulating assembly), US 9,409,017 (describing performing diagnostic testing on implanted devices), and US 9,900,709 (describing determining impedance-related phenomena in a vibrating actuator).

Operation 504 includes enabling automated fitting. In an example, the operation 504 includes the recipient, caregiver, or clinician choosing an automated option for fitting. The automated fitting can be a setting that is enabled on the sensory prosthesis 110. For instance, a flag can be set that enables automated fitting.

Operation 506 includes fitting software creating a map 114. The creating of a map 114 can be, for example, the creation of an initial map 114 for the recipient as part of an initial fitting. In another example, the fitting can be part of a routine fitting process (e.g., a fitting process that typically occurs at checkups of the recipient by the clinician).

Operation 508 includes the clinician going live on the map 114. This operation can include activating the map 114 such that the sensory prosthesis 110 operates with the map 114 being the current map 115. In some examples, the map 114 is first transferred to the sensory prosthesis 110 (see, e.g., operation 512).

Operation 510 includes onboarding the recipient. For example, the onboarding can include providing the sensory prosthesis application 124 to the recipient or the recipient computing device 120. The onboarding can include downloading the sensory prosthesis application 124 from an application distribution platform (e.g., the APPLE APP STORE or GOOGLE PLAY) to the recipient computing device. The operation 510 can further include installing and configuring the sensory prosthesis application 124

Operation 512 includes providing the map 114 to the sensory prosthesis 110. For example, the fitting software transmits the map 114 to sensory prosthesis 110.

Operation 514 includes the application setting the map 114 as the current map 115. For example, the map 114 can be set as map 114 one, such that an indicator of the identifier of the current map 115 (N) is set to one.

Operation 516 includes training the recipient on use of the application. For example, the recipient can be provided with education materials or instruction from the clinician.

Turning to FIG. 5B, Operation 520 includes a trigger occurring. For example, the trigger can be a trigger that causes the performance of automated fitting. Example triggers include a time-based triggers (e.g., the trigger activates once daily, weekly, or monthly or at other intervals), action-based triggers (e.g., responsive to detecting the occurrence of an action, such as the changing of volume or other settings more than a threshold amount of times), or manual triggers (e.g., the clinician or recipient expressly causing the trigger to activate). In an example, the trigger is based on the recipient computing device 120 (e.g., the determination of whether the trigger activates is performed on the recipient computing device 120). In other examples, the trigger is located at another component of the system 100 or at multiple different locations. Responsive to the trigger occurring, the flow of the method 500 can move to operation 522.

Operation 522 includes querying the recipient whether the recipient received uncomfortable stimulation. For example, the user interface 150 is provided by the recipient computing device 120 to provide a query 151 that asks whether the recipient received uncomfortable stimulation. The querying can further include providing one or more user interface elements selectable by the recipient to provide a response. Other input mechanisms can be presented or activated, such as a text box configured to receive text input from a user or by activating a microphone to receive voice input. The operation 522 can further include receiving an answer to the query from the recipient.

Responsive to the response indicating that the recipient received uncomfortable stimulation, the flow of the method 500 can move to operation 524. Responsive to a response indicating that the recipient did not receive uncomfortable stimulation, the flow of the method 500 can move to operation 528.

Operation 524 includes querying the recipient whether the recipient received sounds that were too loud. For example, the user interface 150 is provided by the recipient computing device 120 to provide a query 151 that asks whether the recipient received sounds that were too loud (e.g., the stimulation was too loud because the stimulation was too intense). The querying can further include providing one or more user interface elements selectable by the recipient to provide a response. Other input mechanisms can be presented or activated, such as a text box configured to receive text input from a user or by activating a microphone to receive voice input. The operation 524 can further include receiving an answer to the query from the recipient.

Responsive to the response indicating that the recipient received uncomfortable stimulation, the flow of the method 500 can move to operation 526. Responsive to a response indicating that the recipient did not receive uncomfortable stimulation, it is determined whether the current map 115 is the lowest-loudness map 114 available (e.g., readily available to switch to by the sensory prosthesis 110, such as by being stored on the sensory prosthesis 110 or the recipient computing device 120). For example, the determining can include determining whether the current map 115 is the zero-th map 114 (where maps are indexed from zero and the zero-th map 114 is the softest map). Responsive to the current map 115 being the lowest-loudness map 114 available, the flow of the method moves to operation 534. Responsive to the current loudness map 114 not being the lowest-loudness map 114 available, the flow of the method 500 moves to operation 536.

Operation 526 includes generating a clinician event. For example, the generating of the clinician event can include one or more aspects of operation 260. In some examples, this operation 526 is arrived at because the recipient indicated that the recipient received uncomfortable stimulation that was not too loud. Such a combination of responses may indicate that the recipient is perceiving unintended stimulation (e.g., non-auditory stimulation, where the sensory prosthesis 110 is an auditory prosthesis).

Operation 528 includes querying the recipient regarding softness and loudness. For example, the operation 528 can include providing a query 151 to the user in the form of a user interface 150 that includes a text prompt and user interface elements (e.g., buttons) selectable to obtain input from the recipient. For example, the user interface 150 can be as shown in FIG. 1. The user interface 150 can include a first user interface element 152 selectable to indicate that the stimulation is too loud, a second user interface element 154 selectable to indicate that the stimulation is a little loud, a third user interface element 156 selectable to indicate that the stimulation is just right, a third user interface element 158 selectable to indicate that the stimulation is a little soft, and a fifth user interface element 160 selectable to indicate that the stimulation is too soft. Other implementations of the user interface 150 are also usable. The operation 528 can further include receiving a response from the recipient, such as based on the actuation of one or more of the user interface elements 152, 154, 156, 158, and 160.

Responsive to the response to the query indicating that stimulation is too soft, the flow of the method 500 can move to operation 530. Responsive to the response to the query indicating that stimulation is moderate (e.g., just right or comfortable), the flow of the method 500 can move to a next determination. The next determination can be whether the next loudest map 114 compared to the current map 115 (e.g., the map 114 having an identifier being one greater than the identifier of the current map, where higher identifier numbers are associated with higher loudness) has been tried more than a threshold amount (e.g., more than two times). If so, the flow of the method can move to operation 532. If not, the flow of the method 500.

Operation 530 includes to increment a current map. For example, incrementing the current map 115 can include selecting a next-loudest map 114 as the current map 115. For example, to increment the current map 115 can include to change the current map 115 from the nth map 114 to the (n + 1)th map. The operation 530 can further include incrementing a portion of the log 112 that tracks how many times the (unincremented) map 114 has been tried.

Operation 532 includes to determine that the map 114 is stable. For example, the operation can be reached after determining that the recipient has tried the next loudest map 114 more than a threshold number of times and the recipient indicates that the current stimulation has medium intensity. The operation 532 can include ending a first map phase. For example, the trigger of operation 520 can be disabled until a next map phase begins.

Operation 534 includes to generate a clinician event. For example, the initial map 114 set in the clinic may have been set incorrectly (e.g., too loud). In an example, the operation 534 includes one or more aspects as described in operation 260.

Operation 536 includes to decrement a current map. For example, decrementing the current map 115 can include selecting a next-softest map 114 as the current map. For example, to decrement the current map 115 can include to change the current map 115 from the nth map to the (n - 1)th map. The operation 536 can further include incrementing a portion of the log 112 that tracks how many times a log has been tried.

Turning to FIG. 5C, operation 538 includes to wait for to receive a user input. For example, operation 538 can represent a thread, process, or interrupt that, upon detecting a user input, proceeds with the other operations described in FIG. 5C. In an example, the operation 538 can be non-blocking and the waiting for user input can occur while one or more other operations or processes are being performed. In an example, the operation 538 can be performed while a particular user interface is being presented, such as user interface 150.

Responsive to receiving an input indicating that the current stimulation is too soft, the flow of the method can move to operation 540. Responsive to receiving an input indicating that the current stimulation is too loud, the flow of the method can move to operation 550.

Operation 540 includes to check the log. For example, the log 112 can be checked or analyzed to determine whether the recipient was proximate a loud environment for longer than a threshold amount of time. The operation can include searching or querying the log 112 for entries containing information relating to environments 101 in which the sensory prosthesis 110 was operating. For example, the log 112 can include indications of loudness of signals detected by a microphone or other sensor of the sensory prosthesis 110. Following operation 540, the flow of the method 500 can move to operation 542.

Operation 542 includes to determine whether the recipient was proximate a loud environment for a period of time. For example, the analysis performed in operation 540 can be used to determine whether the recipient was in an environment louder than a threshold for longer than a threshold amount of time.

Operation 544 includes to increment the current map. For example, incrementing the current map 115 can include changing the current map 115 to a different map 114 that has an identifier that is one greater than the identifier of the current map 115 (e.g., in pseudocode, current_map_id = current_map_id + 1).

Operation 546 includes to reset the log 112. In some examples, resetting the log 112 can include deleting one or more entries of the log 112. In some examples, resetting the log 112 can include marking one or more entries of the log 112 as already having been considered or used (e.g., such that the already considered entries are not used for determining whether the user had been in a loud environment as in operation 542). Following operation 546, the flow of the method 500 can move to operation 538.

Operation 548 includes to inform the user of no change. For example, the operation 548 can be performed responsive to the recipient indicating that the sound is too soft but that the user has not been in a loud environment. For example, the user may think that the sound was too soft but in fact the environment was relatively soft. As such, it may be advantageous to avoid making a change to the current map. The user can be informed that no change was made because the recipient was not in a loud environment.

Operation 550 includes to decrement the current map. Decrementing the current map 115 can include changing the current map 115 to a different map 114 that has an identifier that is one (or a different value) less than the identifier of the current map 115. Following operation 548, the flow of the method 500 can move to operation 538.

### Example Devices

As previously described, the technology disclosed herein can be applied in any of a variety of circumstances and with a variety of different devices. For example, the sensory prosthesis 110 can take the form of a variety of different consumer devices or medical devices. Example consumer devices include headphones, earbuds, personal sound amplification products, wireless earbuds, or other consumer devices. Example medical devices include auditory prostheses and visual prostheses. Example auditory prostheses include one or more prostheses selected from the group consisting of: a cochlear implant, an electroacoustic device, a percutaneous bone conduction device, a passive transcutaneous bone conduction device, an active transcutaneous bone conduction device, a middle ear device, a totally-implantable auditory device, a mostly-implantable auditory device, an auditory brainstem implant device, a hearing aid, and a tooth-anchored hearing device. Example visual prostheses include bionic eyes.

Specific example devices that can benefit from technology disclosed herein are described in more detail in FIGS. 6-8, below. For example, the techniques described herein can be used to select broadcasts for medical devices, such as an implantable stimulation system as described in FIG. 6, a cochlear implant as described in FIG. 7, or a retinal prosthesis as described in FIG. 8.

### Example Device - Implantable Stimulator System

FIG. 6 is a functional block diagram of an implantable stimulator system 600 that can benefit from the technologies described herein. In an example, the sensory prosthesis 110 corresponds to the implantable stimulator system 600. The implantable stimulator system 600 includes a wearable device 610 acting as an external processor device and an implantable device 650 acting as an implanted stimulator device. In examples, the implantable device 650 is an implantable stimulator device configured to be implanted beneath a recipient's tissue (e.g., skin). In examples, the implantable device 650 includes a biocompatible implantable housing 602. Here, the wearable device 610 is configured to transcutaneously couple with the implantable device 650 via a wireless connection to provide additional functionality to the implantable device 650.

In the illustrated example, the wearable device 610 includes one or more sensors 620, a memory 111, processor 116, a transceiver 618, and a power source 648. The one or more sensors 620 can be units configured to produce data based on sensed activities. In an example where the stimulation system 600 is an auditory prosthesis system, the one or more sensors 620 include sound input sensors, such as a microphone. Where the stimulation system 600 is a visual prosthesis system, the one or more sensors 620 can include one or more cameras or other visual sensors. The processor 116 can be a component (e.g., a central processing unit) configured to control stimulation provided by the implantable device 650. The stimulation can be controlled based on data from the sensor 620, a stimulation schedule, or other data. Where the stimulation system 600 is an auditory prosthesis, the processor 116 can be configured to convert sound signals received from the sensor(s) 130 (e.g., acting as a sound input unit) into signals 651. The transceiver 618 is configured to send the signals 651 in the form of power signals, data signals, combinations thereof (e.g., by interleaving the signals), or other signals. The transceiver 618 can also be configured to receive power or data. Stimulation signals can be generated by the processor 116 and transmitted, using the transceiver 618, to the implantable device 650 for use in providing stimulation.

In the illustrated example, the implantable device 650 includes a transceiver 618, a power source 648, a coil 656, and a stimulator 640 that includes an electronics module 611 and a stimulator assembly 612. The implantable device 650 further includes a hermetically sealed, biocompatible housing enclosing one or more of the components.

The electronics module 611 can include one or more other components to provide sensory prosthesis functionality. In many examples, the electronics module 611 includes one or more components for receiving a signal (e.g., from one or more of the sensors 620) and converting the signal into the stimulation signal 615. The electronics module 611 can further be or include a stimulator unit. The electronics module 611 can generate or control delivery of the stimulation signals 615 to the stimulator assembly 612. In examples, the electronics module 611 includes one or more processors (e.g., central processing units or microcontrollers) coupled to memory components (e.g., flash memory) storing instructions that when executed cause performance of an operation. In examples, the electronics module 611 generates and monitors parameters associated with generating and delivering the stimulus (e.g., output voltage, output current, or line impedance). In examples, the electronics module 611 generates a telemetry signal (e.g., a data signal) that includes telemetry data. The electronics module 611 can send the telemetry signal to the wearable device 610 or store the telemetry signal in memory for later use or retrieval.

The stimulator assembly 612 can be a component configured to provide stimulation to target tissue. In the illustrated example, the stimulator assembly 612 is an electrode assembly that includes an array of electrode contacts disposed on a lead. The lead can be disposed proximate tissue to be stimulated. Where the system 600 is a cochlear implant system, the stimulator assembly 612 is insertable into the recipient's cochlea. The stimulator assembly 612 can be configured to deliver stimulation signals 615 (e.g., electrical stimulation signals) generated by the electronics module 611 to the cochlea to cause the recipient to experience a hearing percept. In other examples, the stimulator assembly 612 is a vibratory actuator disposed inside or outside of a housing of the implantable device 650 and configured to generate vibrations. The vibratory actuator receives the stimulation signals 615 and, based thereon, generates a mechanical output force in the form of vibrations. The actuator can deliver the vibrations to the skull of the recipient in a manner that produces motion or vibration of the recipient's skull, thereby causing a hearing percept by activating the hair cells in the recipient's cochlea via cochlea fluid motion.

The transceivers 618 can be components configured to transcutaneously receive and/or transmit a signal 651 (e.g., a power signal and/or a data signal). The transceiver 618 can be a collection of one or more components that form part of a transcutaneous energy or data transfer system to transfer the signal 651 between the wearable device 610 and the implantable device 650. Various types of signal transfer, such as electromagnetic, capacitive, and inductive transfer, can be used to usably receive or transmit the signal 651. The transceiver 618 can include or be electrically connected to the coil 656.

The coils 656 can be components configured to receive or transmit a signal 651, typically via an inductive arrangement formed by multiple turns of wire. In examples, in addition to or instead of a coil, other arrangements are used, such as an antenna or capacitive plates. The magnets can be used to align respective coils 656 of the wearable device 610 and the implantable device 650. For example, the coil 656 of the implantable device 650 is disposed in relation to (e.g., in a coaxial relationship) with an implantable magnet set to facilitate orienting the coil 656 in relation to the coil 656 of the wearable device 610 via the force of a magnetic connection. The coil 656 of the wearable device 610 can be disposed in relation to (e.g., in a coaxial relationship) with a magnet set.

The power source 648 can be one or more components configured to provide operational power to other components. The power source 648 can be or include one or more rechargeable batteries. Power for the batteries can be received from a source and stored in the battery. The power can then be distributed to the other components of the implantable device 650 as needed for operation.

### Example Device -Cochlear Implant

FIG. 7 illustrates an example cochlear implant system 710 that can benefit from use of the technologies disclosed herein. For example, the cochlear implant system 710 can be used to implement the sensory prosthesis 110. The cochlear implant system 710 includes an implantable component 744 typically having an internal receiver/transceiver unit 732, a stimulator unit 720, and an elongate lead 718. The internal receiver/transceiver unit 732 permits the cochlear implant system 710 to receive signals from and/or transmit signals to an external device 750. The external device 750 can be a button sound processor worn on the head that includes a receiver/transceiver coil 730 and sound processing components. Alternatively, the external device 750 can be just a transmitter/transceiver coil in communication with a behind-the-ear device that includes the sound processing components and microphone.

The implantable component 744 includes an internal coil 736, and preferably, an implanted magnet fixed relative to the internal coil 736. The magnet can be embedded in a pliable silicone or other biocompatible encapsulant, along with the internal coil 736. Signals sent generally correspond to external sound 713. The internal receiver/transceiver unit 732 and the stimulator unit 720 are hermetically sealed within a biocompatible housing, sometimes collectively referred to as a stimulator/receiver unit. Included magnets can facilitate the operational alignment of an external coil 730 and the internal coil 736 (e.g., via a magnetic connection), enabling the internal coil 736 to receive power and stimulation data from the external coil 730. The external coil 730 is contained within an external portion. The elongate lead 718 has a proximal end connected to the stimulator unit 720, and a distal end 746 implanted in a cochlea 740 of the recipient. The elongate lead 718 extends from stimulator unit 720 to the cochlea 740 through a mastoid bone 719 of the recipient. The elongate lead 718 is used to provide electrical stimulation to the cochlea 740 based on the stimulation data. The stimulation data can be created based on the external sound 713 using the sound processing components and based on sensory prosthesis settings.

In certain examples, the external coil 730 transmits electrical signals (e.g., power and stimulation data) to the internal coil 736 via a radio frequency (RF) link. The internal coil 736 is typically a wire antenna coil having multiple turns of electrically insulated single-strand or multi-strand platinum or gold wire. The electrical insulation of the internal coil 736 can be provided by a flexible silicone molding. Various types of energy transfer, such as infrared (IR), electromagnetic, capacitive and inductive transfer, can be used to transfer the power and/or data from external device to cochlear implant. While the above description has described internal and external coils being formed from insulated wire, in many cases, the internal and/or external coils can be implemented via electrically conductive traces.

### Example Device - Retinal Prosthesis

FIG. 8 illustrates a retinal prosthesis system 800 that comprises an external device 810, a retinal prosthesis 801 and a mobile computing device 803. The retinal prosthesis system 800 can correspond to the sensory prosthesis 110. The retinal prosthesis 800 comprises a processing module 825 and a retinal prosthesis sensor-stimulator 890 is positioned proximate the retina 891 of a recipient. The external device 810 and the processing module 825 can both include transmission coils 856 aligned via respective magnet sets. Signals 851 can be transmitted using the coils 856.

In an example, sensory inputs (e.g., photons entering the eye) are absorbed by a microelectronic array of the sensor-stimulator 890 that is hybridized to a glass piece 892 including, for example, an embedded array of microwires. The glass can have a curved surface that conforms to the inner radius of the retina. The sensor-stimulator 890 can include a microelectronic imaging device that can be made of thin silicon containing integrated circuitry that convert the incident photons to an electronic charge.

The processing module 825 includes an image processor 823 that is in signal communication with the sensor-stimulator 890 via, for example, a lead 888 which extends through surgical incision 889 formed in the eye wall. In other examples, processing module 825 is in wireless communication with the sensor-stimulator 890. The image processor 823 processes the input into the sensor-stimulator 890, and provides control signals back to the sensor-stimulator 890 so the device can provide an output to the optic nerve. That said, in an alternate example, the processing is executed by a component proximate to, or integrated with, the sensor-stimulator 890. The electric charge resulting from the conversion of the incident photons is converted to a proportional amount of electronic current which is input to a nearby retinal cell layer. The cells fire and a signal is sent to the optic nerve, thus inducing a sight perception.

The processing module 825 can be implanted in the recipient and function by communicating with the external device 810, such as a behind-the-ear unit, a pair of eyeglasses, etc. The external device 810 can include an external light / image capture device (e.g., located in / on a behind-the-ear device or a pair of glasses, etc.), while, as noted above, in some examples, the sensor-stimulator 890 captures light / images, which sensor-stimulator is implanted in the recipient.

Similar to the above examples, the retinal prosthesis system 801 may be used in spatial regions that have at least one controllable network connected device associated therewith (e.g., located therein). As such, the processing module 825 includes a performance monitoring engine 827 that is configured to obtain data relating to a "sensory outcome" or "sensory performance" of the recipient of the retinal prosthesis 800 in the spatial region. As used herein, a "sensory outcome" or "sensory performance" of the recipient of a sensory prosthesis, such as retinal prosthesis 800, is an estimate or measure of how effectively stimulation signals delivered to the recipient represent sensor input captured from the ambient environment.

Data representing the performance of the retinal prosthesis 800 in the spatial region is provided to the mobile computing device 803 and analyzed by a network connected device assessment engine 862 in view of the operational capabilities of the at least one controllable network connected device associated with the spatial region. For example, the network connected device assessment engine 862 may determine one or more effects of the controllable network connected device on the sensory outcome of the recipient within the spatial region. The network connected device assessment engine 862 is configured to determine one or more operational changes to the at least one controllable network connected device that are estimated to improve the sensory outcome of the recipient within the spatial region and, accordingly, initiate the one or more operational changes to the at least one controllable network connected device.

### Example Computing System

FIG. 9 illustrates an example of a suitable computing system 900 with which one or more of the disclosed examples can be implemented. Computing systems, environments, or configurations that suitable for use with examples described herein include, but are not limited to, personal computers, server computers, hand-held devices, laptop devices, multiprocessor systems, microprocessor-based systems, programmable consumer electronics (e.g., smart phones), network PCs, minicomputers, mainframe computers, tablets, distributed computing environments that include any of the above systems or devices, and the like. The computing system 900 can be a single virtual or physical device operating in a networked environment over communication links to one or more remote devices. In examples, the sensory prosthesis 110, the recipient computing device 120, the clinician computing device 130, and the fitting server 140 can include one or more components or variations of components of the computing system 900.

In its most basic configuration, computing system 900 includes memory 111 and one or more processors 116. In the illustrated example, the system 900 further includes a network adapter 906, one or more input devices 908, and one or more output devices 910. The system 900 can include other components, such as a system bus, component interfaces, a graphics system, a power source (e.g., a battery), among other components.

The memory 111 is one or more software- or hardware-based computer-readable storage media operable to store information accessible by the one or more processors 116. The memory 111 can store, among other things, instructions executable by the one or more processors 116 to implement applications or cause performance of operations described herein, as well as other data. The memory 111 can be volatile memory (e.g., RAM), non-volatile memory (e.g., ROM), or combinations thereof. The memory 111 can include transitory memory or non-transitory memory. The memory 111 can also include one or more removable or non-removable storage devices. In examples, the memory 111 can include RAM, ROM, EEPROM (Electronically-Erasable Programmable Read-Only Memory), flash memory, optical disc storage, magnetic storage, solid state storage, or any other memory media usable to store information for later access. In examples, the memory 111 encompasses a modulated data signal (e.g., a signal that has one or more of its characteristics set or changed in such a manner as to encode information in the signal), such as a carrier wave or other transport mechanism and includes any information delivery media. By way of example, and not limitation, the memory 111 can include wired media such as a wired network or direct-wired connection, and wireless media such as acoustic, RF, infrared and other wireless media or combinations thereof.

The one or more processors 116 include one or more hardware or software processors. example processors include microprocessors and central processing units. In many examples, the one or more processors 116 are configured to obtain and execute instructions, such as instructions stored in the memory 111. The one or more processors 116 can communicate with and control the performance of other components of the computing system 900.

The network adapter 906 is a component of the computing system 900 that provides network access. The network adapter 906 can provide wired or wireless network access and can support one or more of a variety of communication technologies and protocols, such as ETHERNET, cellular, BLUETOOTH, near-field communication, and RF (Radiofrequency), among others. The network adapter 906 can include one or more antennas and associated components configured for wireless communication according to one or more wireless communication technologies and protocols.

The one or more input devices 908 are devices over which the computing system 900 receives input from a user. The one or more input devices 908 can include physically-actuatable user-interface elements (e.g., buttons, switches, or dials), touch screens, keyboards, mice, pens, and voice input devices, among others input devices.

The one or more output devices 910 are devices by which the computing system 900 can provide output to a user. The output devices 910 can include, displays, speakers, and printers, among other output devices.

As should be appreciated, while particular uses of the technology have been illustrated and discussed above, the disclosed technology can be used with a variety of devices in accordance with many examples of the technology. The above discussion is not meant to suggest that the disclosed technology is only suitable for implementation within systems akin to that illustrated in the figures. In general, additional configurations can be used to practice the processes and systems herein and/or some aspects described can be excluded without departing from the processes and systems disclosed herein.

This disclosure described some aspects of the present technology with reference to the accompanying drawings, in which only some of the possible aspects were shown. Other aspects can, however, be embodied in many different forms and should not be construed as limited to the aspects set forth herein. Rather, these aspects were provided so that this disclosure was thorough and complete and fully conveyed the scope of the possible aspects to those skilled in the art.

As should be appreciated, the various aspects (e.g., portions, components, etc.) described with respect to the figures herein are not intended to limit the systems and processes to the particular aspects described. Accordingly, additional configurations can be used to practice the methods and systems herein and/or some aspects described can be excluded without departing from the methods and systems disclosed herein.

Similarly, where steps of a process are disclosed, those steps are described for purposes of illustrating the present methods and systems and are not intended to limit the disclosure to a particular sequence of steps. For example, the steps can be performed in differing order, two or more steps can be performed concurrently, additional steps can be performed, and disclosed steps can be excluded without departing from the present disclosure. Further, the disclosed processes can be repeated.

Although specific aspects were described herein, the scope of the technology is not limited to those specific aspects. One skilled in the art will recognize other aspects or improvements that are within the scope of the present technology. Therefore, the specific structure, acts, or media are disclosed only as illustrative aspects. The scope of the technology is defined by the following claims.

## Claims

1. A system comprising:
an auditory prosthesis (110) configured to provide stimulation to a recipient to cause auditory percepts based on a current map; and
one or more processors (116) configured to:
receive (310) an indication from the recipient regarding a quality of stimulation; modify (320) the current map (115) based on the quality of stimulation;
determining (330) a difference between the modified current map (115) and a target map (114); and
responsive the difference failing to satisfy a threshold, generate (350) a clinician event.

2. The system of claim 1, wherein to modify (320) the current map based on the quality of the stimulation includes to:
responsive to the quality of the stimulation being too loud, decrement (322) the current map; and
responsive to the quality of the stimulation being too soft, increment (324) the current map.

3. The system of claim 1 or claim 2, wherein the one or more processors (116) are further configured to:
responsive (326) to attempting to decrement the current map beneath a map floor, generate (350) the clinician event; and
responsive (326) to attempting to increment the current map beyond a map ceiling, generate (350) the clinician event.

4. The system of claim 1 or claim 2, further comprising:
a recipient computing device (120) of the recipient communicatively coupled to the auditory prosthesis (110).

5. The system of claim 4, wherein the recipient computing device (120) comprises:
one or more recipient computing device processors (116); and
memory (111) storing fitting instructions that, when executed by the one or more recipient computing device processors, cause the one or more recipient computing device processors (116) to:
query (312) the recipient regarding stimulation provided by the auditory prosthesis (110); and
receive (314) a response to the querying from the recipient.

6. The system of claim 5, wherein the fitting instructions, when executed by the one or more recipient computing device processors (116), further cause the one or more recipient computing device processors (116) to:
modifying (328) the current map of the of the auditory prosthesis based on the received response.

7. The system of claim 5, wherein the fitting instructions, when executed by the one or more recipient computing device processors (116), further cause the one or more recipient computing device processors (116) to:
provide a first user interface element (152) selectable to indicate that the stimulation is too loud; and
provide a second user interface element (156) selectable to indicate that the stimulation is too soft.

8. The system of claim 7, wherein the fitting instructions, when executed by the one or more recipient computing device processors (116), further cause the one or more recipient computing device processors (116) to:
responsive to detecting (362) actuation of the first user interface element (152): determine (364) whether a loud environment was detected proximate the recipient within a threshold amount of time; and
responsive to the loud environment being detected proximate the recipient within the threshold amount of time, decrement (366) the current map; and
responsive to detecting (368) actuation of the second user interface element (156), determine (370) whether a loud environment was detected proximate the recipient within a threshold amount of time; and
responsive to the loud environment being detected proximate the recipient within the threshold amount of time, inform (372) the recipient of no change to the current map.

9. The system of claim 4,
wherein the recipient computing device (120) comprises at least one of the one or more processors (116); or
wherein the recipient computing device (120) is a phone, tablet, or laptop.

10. The system of claim 1 or claim 2, wherein the auditory prosthesis (110) is a cochlear implant;
wherein a server (140) remote from the auditory prosthesis (110) comprises at least one of the one or more processors (116); or
wherein the auditory prosthesis (110) comprises at least one of the one or more processors (H6).

11. A computer-readable medium having instructions (400) stored thereon that, when executed by one or more processors cause the one or more processors to:
after an initial clinical fitting of a sensory prosthesis to a recipient, perform automated fitting of the sensory prosthesis to the recipient, wherein to perform the automated fitting includes to:
query (412) the recipient regarding stimulation provided by the sensory prosthesis;
receive (414) a response to the querying from the recipient; and modifying (416) a map of the sensory prosthesis (110) based on the received response;
determine (420) fitting progress of the automated fitting; and
responsive determining that the fitting progress indicates a lack of progress, generate (430) a clinician event.

12. The computer-readable medium of claim 11, wherein to modify the map includes to:
change (418) the map from a first map (114) stored on the sensory prosthesis to a second map (114) stored on the sensory prosthesis.

13. The computer-readable medium of claim 11 or claim 12, wherein to determine the fitting progress includes to:
determine (422) a fitting trajectory; and
determine (424) whether the fitting trajectory meets a target trajectory.

14. The computer-readable medium of claim 11 or claim 12, wherein to determine the fitting progress includes to:
determine (426) a change in comfort level; and
determine (428) whether the change in comfort level meets a target comfort level.

15. The computer-readable medium of claim 11 or claim 12,
wherein the computer-readable medium is non-transitory;
wherein to generate (432) the clinician event includes alerting the recipient to schedule an appointment for a clinician fitting;
wherein the sensory prosthesis (110) is an auditory prosthesis;
wherein the sensory prosthesis (110) is a visual prosthesis;
wherein the computer-readable medium is a component of a server remote from the sensory prosthesis;
wherein the computer-readable medium is a component of the sensory prosthesis (110); or
wherein the computer-readable medium is a component of a recipient computing device (120) communicatively coupled to the sensory prosthesis (110).

## Patentansprüche

1. System, das Folgendes umfasst:
eine Hörprothese (110), die so konfiguriert ist, dass sie einem Empfänger eine Stimulation bereitstellt, um auf der Grundlage einer aktuellen Karte Hörwahrnehmungen zu erzeugen; und
einen oder mehrere Prozessoren (116), die so konfiguriert sind, dass sie:
eine Angabe vom Empfänger bezüglich der Qualität der Stimulation empfangen (310);
die aktuelle Karte (115) auf der Grundlage der Qualität der Stimulation modifizieren (320);
Bestimmen (330) einer Differenz zwischen der modifizierten aktuellen Karte (115) und einer Zielkarte (114); und
als Reaktion darauf, dass die Differenz einen Schwellenwert nicht erfüllt, Erzeugen (350) eines klinischen Ereignisses.

2. System nach Anspruch 1, wobei das Modifizieren (320) der aktuellen Karte auf der Grundlage der Qualität der Stimulation Folgendes umfasst:
als Reaktion darauf, dass die Qualität der Stimulation zu laut ist, Dekrementieren (322) der aktuellen Karte; und
als Reaktion darauf, dass die Qualität der Stimulation zu leise ist, Inkrementieren (324) der aktuellen Karte.

3. System nach Anspruch 1 oder Anspruch 2, wobei der eine oder die mehreren Prozessoren (116) ferner so konfiguriert sind, dass sie:
als Reaktion (326) auf den Versuch, die aktuelle Karte unter einen Karten-Mindestwert zu verringern, das klinische Ereignis zu generieren (350); und
als Reaktion (326) auf den Versuch, die aktuelle Karte über einen Karten-Höchstwert zu erhöhen, das klinische Ereignis zu generieren (350).

4. System nach Anspruch 1 oder Anspruch 2, das ferner umfasst:
eine Empfänger-Computervorrichtung (120) des Empfängers, das kommunikativ mit der Hörprothese (110) gekoppelt ist.

5. System nach Anspruch 4, wobei die Empfänger-Computervorrichtung (120) umfasst:
einen oder mehrere Empfänger-Computervorrichtungs-Prozessoren (116); und
einen Speicher (111), der Anpassungsanweisungen speichert, die, wenn sie von dem einen oder den mehreren Empfänger-Computervorrichtungs-Prozessoren ausgeführt werden, bewirken, dass der eine oder die mehreren Empfänger-Computervorrichtungs-Prozessoren (116):
den Empfänger bezüglich der von der Hörprothese (110) bereitgestellten Stimulation abfragen (312); und
eine Antwort auf die Abfrage vom Empfänger empfangen (314).

6. System nach Anspruch 5, wobei die Anpassungsanweisungen, wenn sie von dem einen oder den mehreren Empfänger-Computervorrichtungs-Prozessoren (116) ausgeführt werden, den einen oder die mehreren Empfänger-Computervorrichtungs-Prozessoren (116) ferner dazu veranlassen, dass sie:
die aktuelle Karte der Hörprothese auf der Grundlage der empfangenen Antwort modifizieren (328).

7. System nach Anspruch 5, wobei die Anpassungsanweisungen, wenn sie von dem einen oder den mehreren Empfänger-Computervorrichtungs-Prozessoren (116) ausgeführt werden, den einen oder die mehreren Prozessoren (116) der Empfänger-Computervorrichtung ferner dazu veranlassen:
ein erstes Benutzerinterface-Element (152) bereitzustellen, das ausgewählt werden kann, um anzuzeigen, dass die Stimulation zu laut ist; und
ein zweites Benutzeroberflächenelement (156) bereitzustellen, das ausgewählt werden kann, um anzuzeigen, dass die Stimulation zu leise ist.

8. System nach Anspruch 7, wobei die Anpassungsanweisungen, wenn sie von dem einen oder den mehreren Empfänger-Computervorrichtungs-Prozessoren (116) ausgeführt werden, den einen oder die mehreren Empfänger-Computervorrichtungs-Prozessoren (116) ferner dazu veranlassen:
als Reaktion auf die Erkennung (362) der Betätigung des ersten Benutzeroberflächenelements (152): zu bestimmen (364), ob innerhalb einer Schwellenzeitdauer eine laute Umgebung in der Nähe des Empfängers erkannt wurde; und
als Reaktion auf die Erkennung einer lauten Umgebung in der Nähe des Empfängers innerhalb der Schwellenzeitdauer, die aktuelle Karte zu dekrementieren (366); und
als Reaktion auf die Erkennung (368) der Betätigung des zweiten Benutzerschnittstellenelements (156) festzustellen (370), ob innerhalb einer Schwellenzeitdauer eine laute Umgebung in der Nähe des Empfängers erkannt wurde; und
als Reaktion auf die Erkennung einer lauten Umgebung in der Nähe des Empfängers innerhalb der Schwellenzeitdauer den Empfänger darüber zu informieren (372), dass keine Änderung an der aktuellen Karte vorgenommen wurde.

9. System nach Anspruch 4,
wobei die Empfänger-Computervorrichtung (120) mindestens einen der einen oder mehreren Prozessoren (116) umfasst; oder
wobei die Empfänger-Computervorrichtung (120) ein Telefon, ein Tablet oder ein Laptop ist.

10. System nach Anspruch 1 oder Anspruch 2, wobei die Hörprothese (110) ein Cochlea-Implantat ist;
wobei ein von der Hörprothese (110) abgeschiedener Server (140) mindestens einen der einen oder mehreren Prozessoren (116) umfasst; oder
wobei die Hörprothese (110) mindestens einen der einen oder mehreren Prozessoren (H6) umfasst.

11. Computerlesbares Medium mit darauf gespeicherten Anweisungen (400), die, wenn sie von einem oder mehreren Prozessoren ausgeführt werden, den einen oder die mehreren Prozessoren veranlassen:
nach einer ersten klinischen Anpassung einer sensorischen Prothese an einen Empfänger eine automatisierte Anpassung der sensorischen Prothese an den Empfänger durchzuführen, wobei die Durchführung der automatisierten Anpassung Folgendes umfasst:
Abfragen (412) des Empfängers bezüglich der von der sensorischen Prothese bereitgestellten Stimulation;
Empfangen (414) einer Antwort auf die Abfrage vom Empfänger; und
Modifizieren (416) einer Karte der sensorischen Prothese (110) auf der Grundlage der empfangenen Antwort;
Bestimmen (420) des Anpassungsfortschritts der automatisierten Anpassung; und
als Reaktion auf die Feststellung, dass der Anpassungsfortschritt einen Mangel an Fortschritt anzeigt, Erzeugen (430) eines klinischen Ereignisses.

12. Computerlesbare Medium nach Anspruch 11, wobei das Modifizieren der Karte umfasst:
Ändern (418) der Karte von einer ersten Karte (114), die auf der sensorischen Prothese gespeichert ist, zu einer zweiten Karte (114), die auf der sensorischen Prothese gespeichert ist.

13. Computerlesbare Medium nach Anspruch 11 oder Anspruch 12, wobei das Bestimmen des Anpassungsfortschritts umfasst:
Bestimmen (422) einer Anpassungstrajektorien; und
Bestimmen (424), ob die Anpassungstrajektorien einer Zieltrajektorie entspricht.

14. Computerlesbare Medium nach Anspruch 11 oder Anspruch 12, wobei das Bestimmen des Anpassungsfortschritts umfasst:
Bestimmen (426) einer Änderung des Komfortniveaus; und
Bestimmen (428), ob die Änderung des Komfortniveaus einem Zielkomfortniveau entspricht.

15. Computerlesbare Medium nach Anspruch 11 oder Anspruch 12,
wobei das computerlesbare Medium nichtflüchtig ist;
wobei das Erzeugen (432) des klinischen Ereignisses das Benachrichtigen des Empfängers umfasst, einen Termin für eine klinische Anpassung zu vereinbaren;
wobei die sensorische Prothese (110) eine Hörprothese ist;
wobei die sensorische Prothese (110) eine Sehprothese ist;
wobei das computerlesbare Medium eine Komponente eines Servers ist, der von der sensorischen Prothese entfernt ist;
wobei das computerlesbare Medium eine Komponente der sensorischen Prothese (110) ist; oder
wobei das computerlesbare Medium eine Komponente einer Empfänger-Computervorrichtung (120) ist, das kommunikativ mit der sensorischen Prothese (110) gekoppelt ist.

## Revendications

1. Système comprenant :
une prothèse auditive (110) configurée pour fournir une stimulation à un receveur pour entraîner des perceptions auditives sur la base d'une carte de courant ; et
un ou plusieurs processeurs (116) configurés pour :
recevoir (310) une indication de la part du receveur concernant une qualité de stimulation ; modifier (320) la carte de courant (115) sur la base de la qualité de stimulation ;
déterminer (330) une différence entre la carte de courant modifiée (115) et une carte cible (114) ; et
en réponse à la différence tombant pour satisfaire un seuil, générer (350) un évènement clinique.

2. Système selon la revendication 1, dans lequel pour modifier (320) la carte de courant sur la base de la qualité de la stimulation, inclut :
en réponse à la qualité de la stimulation étant trop forte, réduire d'un palier (322) la carte de courant ; et
en réponse à la qualité de la stimulation étant trop légère, augmenter d'un palier (324) la carte de courant.

3. Système selon la revendication 1 ou la revendication 2, dans lequel le un ou plusieurs processeurs (116) sont en outre configurés pour :
en réponse (326) à la tentative de réduire d'un palier la carte de courant en-dessous d'un plancher de carte, générer (350) l'évènement clinique ; et
en réponse (326) à la tentative d'augmenter d'un palier la carte de courant au-delà d'un plafond de carte, générer (350) l'évènement clinique.

4. Système selon la revendication 1 ou la revendication 2, comprenant en outre :
un dispositif de calcul d'un bénéficiaire (120) du receveur communicativement accouplé à la prothèse auditive (110).

5. Système selon la revendication 4, dans lequel le dispositif de calcul d'un bénéficiaire (120) comprend :
un ou plusieurs processeurs de dispositif de calcul d'un bénéficiaire (116) ; et
une mémoire (111) stockant des instructions d'ajustement qui, lorsqu'exécutées par le un ou plusieurs processeurs de dispositif de calcul d'un bénéficiaire, amènent le un ou plusieurs processeurs de dispositif de calcul d'un bénéficiaire (116) à :
questionner (312) le receveur concernant une stimulation fournie par la prothèse auditive (110) ; et
à recevoir (314) une réponse à la requête de la part du receveur.

6. Système selon la revendication 5, dans lequel les instructions d'ajustement, lorsqu'exécutées par le un ou plusieurs processeurs de dispositif de calcul d'un bénéficiaire (116), amènent en outre le un ou plusieurs processeurs de dispositif de calcul d'un bénéficiaire (116) à :
modifier (328) la carte de courant de la de la prothèse auditive sur la base de la réponse reçue.

7. Système selon la revendication 5, dans lequel les instructions d'ajustement, lorsqu'exécutées par le un ou plusieurs processeurs de dispositif de calcul d'un bénéficiaire (116), amènent en outre le un ou plusieurs processeurs de dispositif de calcul d'un bénéficiaire (116) à :
fournir un premier élément d'interface utilisateur (152) pouvant être sélectionné pour indiquer que la stimulation est trop forte ; et
fournir un second élément d'interface utilisateur (156) pouvant être sélectionné pour indiquer que la stimulation est trop légère.

8. Système selon la revendication 7, dans lequel les instructions d'ajustement, lorsqu'exécutées par le un ou plusieurs processeurs de dispositif de calcul d'un bénéficiaire (116), amènent en outre le un ou plusieurs processeurs de dispositif de calcul d'un bénéficiaire (116) à :
en réponse à la détection (362) de l'actionnement du premier élément d'interface utilisateur (152) : déterminer (364) si un environnement bruyant a été détecté près du receveur à l'intérieur d'une quantité de temps seuil ; et
en réponse à l'environnement bruyant qui est détecté près du receveur à l'intérieur de la quantité de temps seuil, réduire d'un palier (366) la carte de courant ; et
en réponse à une détection (368) de l'actionnement du second élément d'interface utilisateur (156), déterminer (370) si un environnement bruyant a été détecté près du receveur à l'intérieur d'une quantité de temps seuil ; et
en réponse à l'environnement bruyant qui est détecté près du receveur à l'intérieur de la quantité de temps seuil, informer (372) le receveur de ne pas changer la carte de courant.

9. Système selon la revendication 4,
dans lequel le dispositif de calcul d'un bénéficiaire (120) comprend au moins l'un du un ou plusieurs processeurs (116) ; ou
dans lequel le dispositif de calcul d'un bénéficiaire (120) est un téléphone, une tablette, ou un ordinateur portable.

10. Système selon la revendication 1 ou la revendication 2, dans lequel la prothèse auditive (110) est un implant cochléaire ;
dans lequel un serveur (140) distant de la prothèse auditive (110) comprend au moins l'un du un ou plusieurs processeurs (116) ; ou
dans lequel la prothèse auditive (110) comprend au moins l'un du un ou plusieurs processeurs (H6).

11. Support lisible par ordinateur ayant des instructions (400) stockées dessus qui, lorsqu'exécutées par un ou plusieurs processeurs amènent le un ou plusieurs processeurs à :
après un ajustement clinique initial d'une prothèse sensorielle à un receveur, effectuer un ajustement automatisé de la prothèse sensorielle au receveur, dans lequel l'exécution de l'ajustement automatisé inclut les étapes consistant à :
questionner (412) le receveur concernant une stimulation fournie par la prothèse sensorielle ;
recevoir (414) une réponse à la requête provenant du receveur ; et modifier (416) une carte de la prothèse sensorielle (110) sur la base de la réponse reçue ;
déterminer (420) un progrès d'ajustement de l'ajustement automatisé ; et
en réponse à une détermination que le progrès d'ajustement indique un manque de progrès, générer (430) un évènement clinique.

12. Support lisible par ordinateur selon la revendication 11, dans lequel la modification de la carte inclut l'étape consistant à :
changer (418) la carte depuis une première carte (114) stockée sur la prothèse sensorielle vers une seconde carte (114) stockée sur la prothèse sensorielle.

13. Support lisible par ordinateur selon la revendication 11 ou la revendication 12, dans lequel la détermination
du progrès d'ajustement inclut les étapes consistant à :
déterminer (422) une trajectoire d'ajustement ; et
déterminer (424) si la trajectoire d'ajustement correspond à une trajectoire cible.

14. Support lisible par ordinateur selon la revendication 11 ou la revendication 12, dans lequel la détermination
du progrès d'ajustement inclut les étapes consistant à :
déterminer (426) un changement dans un niveau de confort ; et
déterminer (428) si le changement dans un niveau de confort correspond à un niveau de confort cible.

15. Support lisible par ordinateur selon la revendication 11 ou la revendication 12,
dans lequel le support lisible par ordinateur est non temporaire ;
dans lequel la génération (432) de l'évènement clinique inclut le fait d'alerter le receveur de programmer un rendez-vous pour un ajustement clinique ;
dans lequel la prothèse sensorielle (110) est une prothèse auditive ;
dans lequel la prothèse sensorielle (110) est une prothèse visuelle ;
dans lequel le support lisible par ordinateur est un composant d'un serveur distant de la prothèse sensorielle ;
dans lequel le support lisible par ordinateur est un composant de la prothèse sensorielle (110) ; ou
dans lequel le support lisible par ordinateur est un composant d'un dispositif de calcul d'un bénéficiaire (120) accouplé de manière à pouvoir communiquer à la prothèse sensorielle (110).
